# EUROPÄISCHE PATENTSCHRIFT

(11) **EP 0 365 621 B1**
(45) Veröffentlichungstag und Bekanntmachung des Hinweises auf die Patenterteilung: **18.01.1995**
(21) Anmeldenummer: 89903676.8
(22) Anmeldetag: 04.04.1989
(51) Int. Cl.: C12M 3/00

(54) **VORRICHTUNG ZUM SUBMERSEN KULTIVIEREN VON GEWEBEZELLEN**
DEVICE FOR SUBMERGED CULTURE OF TISSUE CELLS
DISPOSITIF DE CULTURE SUBMERGEE DE CELLULES TISSULAIRES

(30) Priorität: 05.04.1988 CH 1244/88
(43) Veröffentlichungstag der Anmeldung: 02.05.1990
(73) Patentinhaber: KLOSS, Gerd, D-83059 Kolbermoor (DE)
(72) Erfinder: Kloss, Gerd, D-8208 Kolbermoor (DE)
(74) Vertreter: Bosshard, Ernst
(86) Internationale Anmeldenummer: CH8900067
(87) Internationale Veröffentlichungsnummer: WO8909814

(56) Entgegenhaltungen:
- EP-A- 0 095 804
- DE-A- 3 313 081
- FR-A- 765 295
- CHEMICAL ENGINEERING, vol. 81, no. 6, 18 March 1974; 'Rotary sparger features porous-metal impeller', page 80, see the whole article

## Beschreibung

Die Erfindung betrifft eine Vorrichtung zum submersen Kultivieren von pflanzlichen, tierischen una humanen Gewebezellen in einem geschlossenen Raum, bestehend aus einem sterilisierbaren Behälter mit einer zentral angeordneten, rotierbaren Hohlwelle mit einem oder mehreren übereinander angeordneten Rührelementen und Schikanen.

Bei der submersen Kultivierung von Gewebezellen haben sich die bekannten in der Züchtung von Mikroorganismen gebräuchlichen Rührer und Mischer wegen der auftretenden Scherkräfte nicht bewährt. Vor allem bei der Kultivierung von menschlichen Zellinien führten die starken mechanischen Bewegungen zu untolerierbaren Zellschädigungen. Man ist deshalb dazu übergegangen, den Nährstoff- und Gasaustausch im sog. Airliftverfahren durchzuführen, indem mittels Begasung durch Lochplatten das Belüftungsmedium für die erforderliche Durchmischung sorgte. Auch eine Kombination von pneumatischer und mechanischer Durchmischung wurde versucht.

So beschreibt die EP-A-0 095 804 ein Verfahren zur submersen Züchtung von Gewebezellen, wobei ein relativ langsam laufendes Rührwerk für eine Durchmischung von Nährstoffen und Luft und eine im Boden des Behälters vorgesehene Sinterplatte für die Begasung sorgt. Die bekannte Vorrichtung hat den Nachteil dass kein ausreichender Gastransfer zur und von der Zelle erfolgen kann, da eine Begasung in einem engen Bereich nur vom Boden des Behälters aus erfolgen kann. Durch das relativ langsam laufende Rührwerk erfolgt eine mangelhafte Belüftung im Bereich sowohl der Behälterwände als auch im oberen Teil des Behälters.

Aufgabe der Erfindung ist es, eine Vorrichtung zu schaffen, welche eine optimale Begasung in einem Kulturgefäss gestattet und eine Zellschädigung vermeidet.

Die Aufgabe wird erfindungsgemäss dadurch gelöst, dass die Schikanen zwischen je zwei Rührelementen angeordnet sind, welche mit einem gasundurchlässigen Teil und einem gasdurchlässigen Teil versehen sind.

Eine solche Vorrichtung hat den Vorteil, dass eine Begasung mit höchster Intensität, trotz langsam laufenden Rührwerk, in verschiedenen Ebenen erfolgen kann, und zwar bis zur Oberfläche der Flüssigkeit. Die bausteinartige Zusammensetzung der Rührelemente auf der Hohlwelle ermöglicht den Einbau in Behälter mit verschiedenen Dimensionen, abhängig von der Art der zu züchtenden Zellen. Durch die hohlen Rührelemente ist so eine optimale Begasung in jeder Höhe gewährleistet, ohne dass Scherkräfte durch ein schneller laufendes Rührwerk eine Zellschädigung hervorrufen können.

Es ist besonders zweckmässig, den oberen Teil der Rührelemente aus einem Blech herzustellen, wobei sich veredelter, rostfreier Stahl als besonders vorteilhaft erwiesen hat. Die Gasöffnungen können rund sein oder eine andere zweckmässige Geometrie aufweisen.

Ein weiteres Verfahren (FR-A-765 295), insbesondere zur Herstellung von Hefe benutzt als Belüftungsmittel hohle Drehflügel mit Belüftungsbohrungen. Zusätzlich sind in einer einzigen Ebene schikanenartige Führungsplatten unterhalb der Drehflügel vorgesehen. Oberhalb der Drehflügel befindet sich ein rohrförmiges Kühlsystem. Eine optimale Durchmischung und Begasung von technischen Fermentationsanlagen für scheerempflindliche Zellen ist nach diesem Verfahren und mit diesen Mitteln nicht moglich.

Es ist zweckmässig, den oberen Teil der Rührelemente als porösen Körper zu gestalten, während der untere Teil geschlossen ist. Es kann aber auch des gesamte Rührelement aus einem porösen Material bestehen.

Als poröses Material sind Sintermetall, Keramik und Kunststoff geeignet. Ein Kunststoff muss jedoch so thermostabil sein, dass er zahlreiche Sterilisationen bei etwa 130° C ohne Schädigung übersteht.

Es hat sich aber auch für bestimmte Zellzüchtungen als zweckmässig erwiesen, den gasdurchlässigen Teil der Schikanen mit einer semipermeablen Membran zu versehen. Eine solche Membran erlaubt neben dem Gastransfer auch eine Zufuhr von Nährmedium.

Es hat sich ferner als zweckmässig erwiesen, die Schikanen zwischen einem Rührelementpaar innerhalb eines herausnehmbaren Schikanenkorbes anzuordnen. Der Schikanenkorb kann im Bedarfsfall separat gereinigt und vorsterilisiert werden. Es können so grossflächige, langsam laufende Rührwerke, die an der Oberseite Gasdurchlässe aufweisen, in einem Schikanenkorb rotieren.

Es ist von Vorteil, wenn auch die Schikanen aus einem Hohlkörper bestehen und im Bedarfsfall zur Verstärkung einer Begasung herangezogen werden können. In diesem Fall bestehen die Schikanen gleichfalls, wie die Rührelemente, aus einem gasundurchlässigen und einem gasdurchlässigen Hohlkörper.

Die Erfindung soll anhand einer Zeichnung näher erläutert werden.
Es zeigen:
- Fig. 1: eine Anordnung der erfindungsgemässen Rührelemente mit einem Schikanenkorb im unteren Teil eines Behälters
- Fig. 2: einen Schnitt durch den Behälter im Bereich A - A der Fig. 1
- Fig. 3: Detail eines Rührelementes

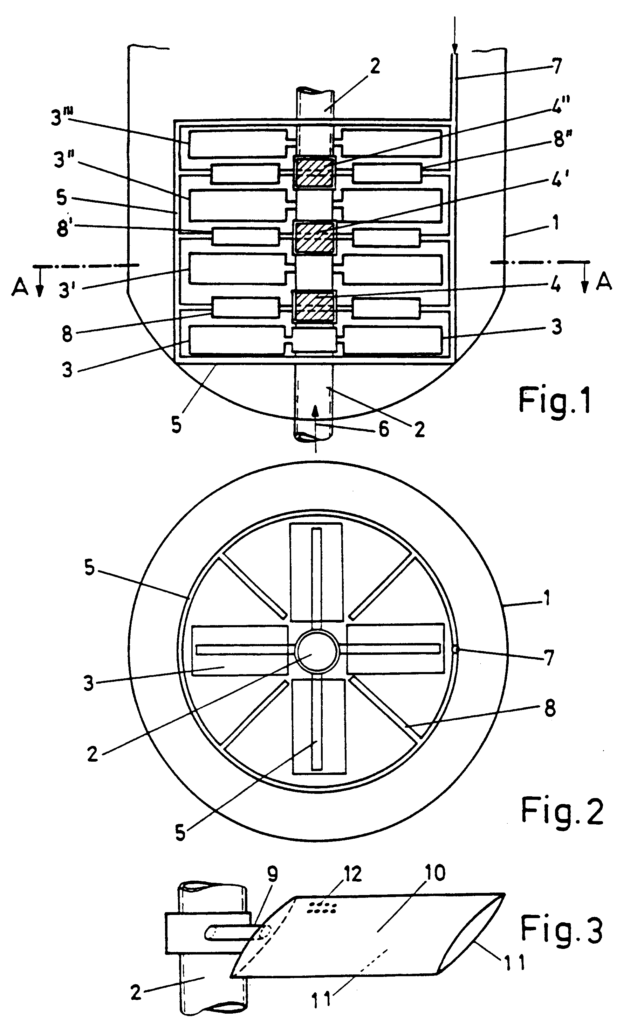

In Figur 1 ist der untere Teil eines Behälters 1 mit einer Hohlwelle 2 gezeigt. Die Hohlwelle ist in bekannter Weise in ihrem unteren Teil durch die Behälterwand hindurchgeführt und abgedichtet. Die Hohlwelle 2 ist von unten mit einem nicht gezeigten Elektromotor und einer Gaszufuhr 6 verbunden. Auf der Hohlwelle 2 sind paaweise gestaltete Rührelemente 3, 3', 3'' und 3''' angeordnet, die durch Distanzringe 4, 4', 4'' voneinander getrennt sind. Ein Schikanenkorb 5 ist im Behälter 1 so angeordnet, dass er die Rührelemente 3 und die Schikanen 8 umschliesst. Am Schikanenkorb 8 ist ein Anschluss 7 für ein Druckgas vorgesehen.

In Figur 2 sind im Behälter 1 die Rührflügelpaare 3 innerhalb des Schikanenkorbes 5 angeordnet. Anstelle eines Rührelementes mit vier Flügeln können auch zwei Flügel vorgesehen werden . Im Inneren des Rührelementes 3 sind Luftkanäle 9 vorgesehen, welche mit der Hohlwelle 2 in Verbindung stehen. Auch die Schikanen 8 können hohl ausgebildet sein und mit dem hohlen Schikanenkorb 5 verbunden sein, welcher seinerseits mit dem Anschluss 7 für die Gaszufuhr verbunden ist.

In Figur 3 ist das Rührelement 3 perspektivisch dargestellt. Es zeigt einen gasdurchlässigen obere Teil 10 mit Gasaustrittsöffnungen 12 und einen geschlossenen unteren Teil 11. Im Innern des Rührelementes 3 ist gestrichelt der Luftkanal 9 gezeichnet, der mit der Hohlwelle 2 verbunden ist.

Im Betrieb ist der Behälter 1 mit einem Nährmedium und Gewebezellen versehen. Die Hohlwelle 2 wird mit einer Geschwindigkeit bis höchstens 30 Umdrehungen (Upm), bevorzugt mit 5 bis 15 Upm bewegt. Gleichzeitig wird Druckluft über die Gaszufuhr 6 und die Hohlwelle 2 den hohlen Rührelementen 3 zugeführt. Bevorzugt befindet sich der Gasaustritt an der Oberseite der Rührelemente 3. Durch die zwischen den Rührelementen 3 im Schikanenkorb 5 angeordneten Schikanen 8 wird der Rühreffekt gewährleistet. Für einen erhöhten Gasbedarf kann Druckluft über den Anschluss 7 und den hohlen Schikanenkorb 5 den Schikanen 8 zugeführt werden, welche in diesem Fall gleichfalls Austrittsöffnungen für das Gas aufweisen.

Anstelle einer gelochten, perforierten oder porösen Oberseite 10 der Rührelemente 3 kann eine semipermeable Membran vorgesehen werden. Die Zufuhr vom Medium kann dann durch die Hohlwelle 2 in die Rührelemente 3 erfolgen. Auf diese Weise können die Rührelemente 3 für einen intensiven Stoffaustausch eingesetzt werden. Auch die Schikanen 8 können zur Verstärkung des Stoffaustausches mit den genannten Membranen vorgesehen werden. In diesem Fall erfolgt die Mediumzufuhr über den hohlen Schikanenkorb 5.

Die erfindungsgemässe Vorrichtung ist besonders zum Züchten scherempfindlicher Zellen und Zellinien geeignet und durch die wechselweise Anordnung von hohlen Rührelementen und Schikanen weitgehend von der Geometrie des Behälters unabhängig. Je nach Bedarf können mehr oder weniger Rührelemente in einfacher Weise übereinander angeordnet werden. Ein weiterer Vorteil ist darin zu sehen, dass die Medienzufuhr gleichzeitig über die Begasungsleitung erfolgen kann.

Die Vorrichtung ist neben Gewebezellen auch zur submersen Kultivierung von Mikroorganismen aller Art geeignet, insbesonders von solchen, welche scherempfindlich sind.

## Patentansprüche

1. Vorrichtung zum submersen Kultivieren von pflanzlichen, tierischen und humanen Gewebezellen in einem geschlossenen Raum, bestehend aus einem sterilisierbaren Behälter (1) mit einer zentral angeordneten Hohlwelle (2) mit mindestens einer Schikane zwischen zwei Rührelementen und/oder mit mehreren übereinander angeordneten Rührelementen (3) und Schikanen (8), dadurch gekennzeichnet, dass die Schikanen (8) jeweils zwischen zwei Rührelementen (3) angeordnet sind, welche Rührelemente (3) mit einem gasundurchlässigen Teil (11) und einem gasdurchlässigen Teil (10) versehen sind.

2. Vorrichtung nach Anspruch 1, dadurch gekennzeichnet, dass der gasdurchlässige Teil (10) aus einem gelochten Blech besteht.

3. Vorrichtung nach Anspruch 1, dadurch gekennzeichnet, dass der gasdurchlässige Teil (10) aus einem porösen Material besteht.

4. Vorrichtung nach Anspruch 3, dadurch gekennzeichnet, dass das poröse Material ein Sintermaterial, Keramik oder ein Kunststoff ist.

5. Vorrichtung nach Anspruch 1, dadurch gekennzeichnet, dass das gasdurchlässige Material eine semipermeable Membran ist.

6. Vorrichtung nach Anspruch 1, dadurch gekennzeichnet, dass die Schikanen (8) an einem herausnehmbaren Schikanenkorb (5) befestigt sind.

7. Vorrichtung nach Anspruch 1, dadurch gekennzeichnet, dass die Schikanen (8) und der Schikanenkorb (5) als Hohlkörper ausgebildet sind.

8. Vorrichtung nach Anspruch 7, dadurch gekennzeichnet, dass ein Teil der Schikanen (8) gasdurchlässig ausgebildet ist.

## Claims

1. Device for submerged culture of vegetable, animal or humane tissue cells in a closed room, consisting of a sterilizable container (1) with a central positioned hollow shaft (2) with at least one vexation between two agitator elements and/or with several superposed agitator elements (3) and vexations (8), characterised in that each of the vexations (8) is arranged between two agitator elements (3), which agitator elements (3) are provided with a gastight portion (11) and a gas permeable portion (10).

2. Device as claimed in claim 1, characterised in that the gas permeable portion (10) consisting of a perforated sheet.

3. Device as claimed in claim 1, characterised in that the gas permeable portion (10) consisting of a porous material.

4. Device as claimed in claim 3, characterised in that the porous material is a sintered material, ceramic or a plastic material.

5. Device as claimed in claim 1, characterised in that the gas permeable material is a semipermeable membrane.

6. Device as claimed in claim 1, characterised in that the vexations (8) are fastened at a removable vexation basket (5).

7. Device as claimed in claim 1, characterised in that the vexations (8) and the vexation basket (5) are formed as hollow body.

8. Device as claimed in claim 7, characterised in that a part of the vexations (8) is formed gas permeable.

## Revendications

1. Dispositif de culture submergée de cellules tissulaires végétales, animales ou humaines en milieu clos, constitué d'un récipient (1) stérilisable avec un arbre creux (2) en position centrale avec au moins une chicane entre deux agitateurs et/ou avec plusieurs agitateurs (3) et chicanes (8) disposés les uns au-dessus des autres, caractérisé en ce que les chicanes (8) sont placées chacune entre deux agitateurs (3), lesquels agitateurs (3) comprennent une partie (11) imperméable aux gaz et une partie (10) perméable aux gaz.

2. Dispositif selon la revendication 1, caractérisé en ce que la partie (10) perméable aux gaz est constituée d'une tôle perforée.

3. Dispositif selon la revendication 1, caractérisé en ce que la partie (10) est constituée d'un matériau poreux.

4. Dispositif selon la revendication 3, caractérisé en ce que le matériau poreux est un matériau fritté, une céramique ou une matière plastique.

5. Dispositif selon la revendication 1, caractérisé en ce que le matériau perméable aux gaz est une membrane semiperméable.

6. Dispositif selon la revendication 1, caractérisé en ce que les chicanes (8) sont fixées sur un panier à chicanes (5) démontable.

7. Dispositif selon la revendication 1, caractérisé en ce que les chicanes (8) et le panier à chicanes (5) sont creux.

8. Dispositif selon la revendication 7, caractérisé en ce qu'une partie des chicanes (8) est perméable aux gaz.
